# EUROPÄISCHE PATENTSCHRIFT

(11) **EP 2 913 314 B1**
(45) Veröffentlichungstag und Bekanntmachung des Hinweises auf die Patenterteilung: **23.11.2016**
(21) Anmeldenummer: 15162943.3
(22) Anmeldetag: 07.07.2011
(51) Int. Cl.: C03C 10/12, C03C 3/095, A61K 6/027

(54) **VERFAHREN ZUR HERSTELLUNG VON DENTALRESTAURATIONEN**
METHOD FOR PRODUCING DENTAL RESTORATIONS
PROCÉDÉ DE FABRICATION DE RESTAURATIONS DENTAIRES

(30) Priorität: 07.07.2010 EP 10168792; 18.04.2011 EP 11162840
(43) Veröffentlichungstag der Anmeldung: 02.09.2015
(62) Teilanmeldung aus: 11173131.1
(73) Patentinhaber: Ivoclar Vivadent AG, 9494 Schaan (LI)
(72) Erfinder: Ritzberger, Christian, 9472 Grabs (CH); Höland, Wolfram, 9494 Schaan (LI); Schweiger, Marcel, 7000 Chur (CH); Rheinberger, Volker, 9490 Vaduz (LI)
(74) Vertreter: Uexküll & Stolberg

(56) Entgegenhaltungen:
- EP-A1- 0 690 031
- EP-A1- 1 688 398
- WO-A1-2009/126317
- DE-A1-102005 026 269

## Beschreibung

Die Erfindung betrifft ein Verfahren zur Herstellung von Dentalrestaurationen, bei dem Lithiumsilikat-Glaskeramiken und -Gläser mit hohem Gehalt an einem Element mit hoher Ordnungszahl eingesetzt werden.

Lithiumsilikat-Glaskeramiken zeichnen sich durch sehr gute mechanische Eigenschaften aus, weshalb sie seit langem im Dentalbereich und dort vornehmlich zur Herstellung von Dentalkronen und kleinen Brücken Anwendung finden. Die bekannten Lithiumsilikat-Glaskeramiken enthalten üblicherweise als Hauptkomponenten SiO₂, Li₂O, Al₂O₃, Alkalimetalloxide wie Na₂O oder K₂O und Keimbildner wie P₂O₅. Darüber hinaus können sie als weitere Komponenten beispielsweise weitere Alkalimetalloxide und/oder Erdalkalimetalloxide und/oder ZnO enthalten. Es sind auch Glaskeramiken bekannt, die in geringen Mengen weitere Metalloxide und insbesondere färbende und fluoreszierende Metalloxide enthalten.

EP 1 505 041 beschreibt Lithiumsilikat-Glaskeramiken, die zusätzlich 0 bis 2 Gew.-% ZrO₂ sowie 0,5 bis 7,5 Gew.-% und insbesondere 0,5 bis 3,5 Gew.-% färbende und fluoreszierende Metalloxide enthalten können. EP 1 688 398 beschreibt ähnliche Lithiumsilikat-Glaskeramiken, die im Wesentlichen frei von ZnO sind und neben färbenden und fluoreszierenden Metalloxiden in den vorgenannten Mengen auch 0 bis 4 Gew.-% ZrO₂ enthalten können, wobei zur Erzielung hoher Festigkeiten allerdings geringere Mengen von 0 bis 2 Gew.-% ZrO₂ bevorzugt sind. Die Glaskeramiken werden insbesondere in Form der Lithiummetasilikat-Glaskeramiken mittels CAD/CAM-Verfahren zu den gewünschten Dentalrestaurationen verarbeitet, wobei eine anschließende Wärmebehandlung die Umwandlung der Metasilikat-Phase in die hochfeste Disilikat-Phase bewirkt.

US 6,455,451 betrifft Lithiumdisilikat-Glaskeramiken, die neben anderen Komponenten auch Übergangsmetalloxide enthalten können. Dabei wird unter anderem vorgeschlagen, zur Erhöhung des Brechungsindex der Glasmatrix kleine Mengen schwerer Elemente wie Sr, Y, Nb, Cs, Ba, Ta, Ce, Eu oder Tb zuzugeben. So können beispielweise CeO₂ und Tb₄O₇ in Mengen 0 bis 1 Gew.-%, Nb₂O₃ und Ta₂O₅ in Mengen von 0 bis 2 Gew.-% und ZrO₂ und Y₂O₃ in Mengen von 0 bis 3 Gew.-% verwendet werden. In einer Ausführungsform soll Ta₂O₅ in einer Menge von 0,5 bis 8 Gew.-% vorliegen können, wobei die konkreten Beispiele allerdings maximal 2,02 Gew.-% dieses Oxids aufweisen.

US 5,176,961 und US 5,219,799 offenbaren Glaskeramiken beispielsweise zur Herstellung von Geschirr, die als Färbemittel bestimmte Übergangsmetalloxide wie CeO₂, Co₃O₄, Cr₂O₃, CuO, Fe₂O₃, MnO₂, NiO und V₂O₅ in einer Menge von 0,01 bis 7 Gew.-% enthalten können.

US 5,507,981 und US 5,702,514 beschreiben Verfahren zur Formung von Dentalrestaurationen und in diesen Verfahren einsetzbare Glaskeramiken. Dabei handelt es sich insbesondere um Lithiumdisilikat-Glaskeramiken, die 0 bis 5 Gew.-% färbende Oxide wie SnO₂, MnO, CeO, Fe₂O₃, Ni₂O, V₂O₃, Cr₂O₃ oder TiO₂ enthalten können.

Bekannte Glaskeramiken auf Basis von Lithiumsilikat weisen häufig optische Eigenschaften auf, die den ästhetischen Anforderungen insbesondere im Zusammenhang mit der Verwendung als Dentalmaterialien nicht in ausreichendem Maße genügen. So weisen bekannte Glaskeramiken oft einen ungünstigen Brechungsindex auf. Insbesondere besteht bei Glaskeramiken das Problem, dass die Brechungsindizes der kristallinen Phase und der Glasphase üblicherweise deutlich voneinander abweichen, was eine in den meisten Fällen unerwünschte Eintrübung der Glaskeramik zur Folge hat. Ähnliche Probleme bestehen etwa im Falle von Kompositen, weil die Brechungsindizes bekannter Glaskeramiken und Gläser üblicherweise von denen der Polymerphase abweichen. Es besteht daher ein Bedarf an Glaskeramiken auf Basis von Lithiumsilikat, deren Brechungsindex in einfacher Weise variiert werden kann, jedoch ohne dass dabei die sonstigen Eigenschaften wesentlich beeinträchtigt werden. Es ist zudem wünschenswert, dass solche Glaskeramiken unter vergleichbaren Bedingungen wie übliche Glaskeramiken hergestellt und kristallisiert werden können und aus ihnen in vorteilhafter Weise Dentalrestaurationen, wie Inlays oder Kronen, hergestellt werden können.

Der Erfindung liegt die Aufgabe zugrunde Verfahren zur Herstellung von Dentalrestaurationen zur Verfügung zu stellen, bei denen Glaskeramiken und/oder Gläser eingesetzt werden können, die die vorstehend geschilderten Nachteile vermeiden und sich vorteilhaft zu den jeweils gewünschten Restaurationen verarbeiten lassen.

Diese Aufgabe wird durch das Verfahren nach einem der Ansprüche 1 bis 11 gelöst. Gegenstand der Erfindung ist ebenfalls die Verwendung nach Anspruch 12.

Das erfindungsgemäße Verfahren zur Herstellung von Dentalrestaurationen zeichnet sich dadurch aus, dass eine Lithiumsilikat-Glaskeramik oder ein Lithiumsilikat-Glas durch
(i) Verpressen oder
(ii) maschinelle Bearbeitung
zur Dentalrestauration verformt wird, wobei die Glaskeramik und das Glas mindestens 8,5 Gew.-% Übergangsmetalloxid ausgewählt aus der Gruppe bestehend aus Oxiden von Y, Nb, La, Ta, W und Mischungen dieser Oxide enthalten und wobei die Glaskeramik Lithiummetasilikat oder Lithiumdisilikat als Hauptkristallphase aufweist und das Glas Keime enthält, die zur Ausbildung von Lithiummetasilikat- und/oder Lithiumdisilikatkristallen geeignet sind.

Bei den hergestellten Dentalrestaurationen handelt es sich bevorzugt um Inlays, Onlays, Kronen, Veneers, Schalen oder Abutments.

Vorzugsweise sind Dentalrestaurationen ausgenommen, die ein Verbund sind, in dem Zirkonoxidkeramik mit der Lithiumsilikat-Glaskeramik oder dem Lithiumsilikat-Glas beschichtet ist. Besonders bevorzugt sind Dentalrestaurationen ausgenommen, die einen solchen Verbund aufweisen.

Die Dentalrestauration ist vorzugsweise auf Basis von Lithiumdisilikat-Glaskeramik, da diese über ausgezeichnete optische und mechanische Eigenschaften verfügt.

Die Glaskeramik und das Glas werden im erfindungsgemäßen Verfahren vorzugsweise in Form von Rohlingen, wie Blöcken oder Zylindern, eingesetzt.

Das Verpressen oder die maschinelle Bearbeitung der Glaskeramik oder des Glases geben diesen die Form der gewünschten Dentalrestauration. Vorzugsweise umfasst das Verpressen nicht deren Aufpressen auf einen anderen Werkstoff, wie z.B. Zirkonoxidkeramik. Ein solches Aufpressen dient zur Beschichtung dieses anderen Werkstoffes.

Das Verpressen erfolgt üblicherweise bei erhöhter Temperatur und erhöhtem Druck. Es ist bevorzugt, dass das Verpressen bei einer Temperatur von 700 bis 1200°C erfolgt. Weiter ist es bevorzugt, dass das Verfahren bei einem Druck von 2 bis 10 bar erfolgt. Beim Verpressen kommt es infolge von viskosem Fließen des eingesetzten Materials zu einer Formänderung.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens erfolgt. Weiter ist es bevorzugt, nach der maschinellen Bearbeitung mindestens eine Wärmebehandlung durchzuführen, um das verformte Glas oder die verformte Glaskeramik zu Lithiumdisilikat-Glaskeramik umzuwandeln.

Die erfindungsgemäß eingesetzte Lithiumsilikat-Glaskeramik zeichnet sich dadurch aus, dass sie mindestens 8,5 Gew.-% Übergangsmetalloxid ausgewählt aus der Gruppe bestehend aus Oxiden von Y, Nb, La, Ta, W und Mischungen dieser Oxide enthält.

Generell ist es bevorzugt, dass das Übergangsmetalloxid als Komponente der erfindungsgemäß eingesetzten Glaskeramik oder des erfindungsgemäß eingesetzten Glases im Wesentlichen keine Farbänderung gegenüber einer entsprechenden Glaskeramik bzw. einem entsprechenden Glas ohne Zusatz dieser Komponente bewirkt. Insbesondere ist das Übergangsmetalloxid farblos und/oder nichtfluoreszierend.

Dabei sind Glaskeramiken bevorzugt, die 8,5 bis 30,0 Gew.-%, vorzugsweise 9,0 bis 25,0 Gew.-%, insbesondere 9,5 bis 20,0 Gew.-%, bevorzugt 10,0 bis 18,0 Gew.-%, weiter bevorzugt 10,5 bis 16,0 Gew.-% und am meisten bevorzugt 11,0 bis 15,0 Gew.-% Übergangsmetalloxid ausgewählt aus einer oder mehreren der vorgenannten Gruppen enthalten.

Überraschenderweise lässt sich durch die Verwendung des erfindungsgemäßen hohen Gehalts an Übergangsmetall mit hoher Ordnungszahl der Brechungsindex von Glaskeramiken und Gläsern auf Basis von Lithiumsilikat in einfacher Weise einstellen, ohne dass andere Eigenschaften wesentlich beeinträchtigt werden. Insbesondere hat sich unerwartet gezeigt, dass regelmäßig der hohe Gehalt an Übergangsmetall mit hoher Ordnungszahl weder die gewünschte Kristallisation von Lithiumdisilikat verhindert, noch zur Bildung unerwünschter Nebenkristallphasen führt, so dass erfindungsgemäß Glaskeramiken mit ausgezeichneten optischen und mechanischen Eigenschaften erhalten werden.

Bevorzugt ist weiter eine Glaskeramik, die 54,0 bis 80,0 und insbesondere 60,0 bis 70,0 Gew.-% SiO₂ enthält.

Außerdem ist eine Glaskeramik bevorzugt, die 11,0 bis 19,0 und insbesondere 12,0 bis 15,0 Gew.-% Li₂O enthält.

Es hat sich als besonders bevorzugt erwiesen, wenn die Glaskeramik 0,5 bis 12,0 und insbesondere 2,5 bis 6,0 Gew.-% Keimbildner enthält. Bevorzugte Keimbildner sind ausgewählt aus P₂O₅, TiO₂, Metallen, z.B. Pt, Pd, Au, Ag oder Mischungen davon. Besonders bevorzugt enthält die Glaskeramik P₂O₅ als Keimbildner. Überraschenderweise bewirkt insbesondere P₂O₅ als Keimbildner die Ausbildung von gewünschten Lithiumdisilikat-Kristallen und vermeidet auf der anderen Seite weitgehend die Bildung unerwünschter Nebenkristallphasen.

Die erfindungsgemäß eingesetzte Glaskeramik enthält bevorzugt weiteres Alkalimetalloxid in einer Menge von 0,5 bis 13,0, bevorzugt 1,0 bis 7,0 und besonders bevorzugt 2,0 bis 5,0 Gew.-%. Der Begriff "weiteres Alkalimetalloxid" bezeichnet Alkalimetalloxid mit Ausnahme von Li₂O. Das weitere Alkalimetalloxid ist insbesondere K₂O, Cs₂O und/oder Rb₂O und ist besonders bevorzugt K₂O. Es wird angenommen, dass der Einsatz von K₂O gegenüber dem in konventionellen Glaskeramiken eingesetzten Na₂O zur Verstärkung des Glasnetzwerkes beiträgt. Es ist bevorzugt, dass die Glaskeramik weniger als 2,0, insbesondere weniger als 1,0, bevorzugt weniger als 0,5 und besonders bevorzugt im Wesentlichen kein Na₂O enthält.

Weiter ist es bevorzugt, dass die Glaskeramik bis zu 6,0 Gew.-% und insbesondere 0,1 bis 5,0 Gew.-% Erdalkalimetalloxid enthält, wobei das Erdalkalimetalloxid insbesondere CaO, BaO, MgO, SrO oder eine Mischung davon ist.

Weiterhin ist es bevorzugt, dass die Glaskeramik bis zu 6,0 Gew.-% und insbesondere 0,1 bis 5,0 Gew.-% ZnO enthält.

Die erfindungsgemäß eingesetzte Glaskeramik kann darüber hinaus noch Zusatzkomponenten enthalten, die insbesondere ausgewählt sind aus Oxiden dreiwertiger Elemente, weiteren Oxiden vierwertiger Elemente, weiteren Oxiden fünfwertiger Elemente, Schmelzbeschleunigern, Färbemitteln und Fluoreszenzmitteln.

Bevorzugt ist eine Glaskeramik, die 0,2 bis 8,0, insbesondere 1,0 bis 7,0 und bevorzugt 2,5 bis 3,5 Gew.-% Oxid dreiwertiger Elemente enthält, wobei dieses Oxid insbesondere ausgewählt ist aus Al₂O₃, Bi₂O₃ und Mischungen davon, und bevorzugt Al₂O₃ ist.

Der Begriff "weitere Oxide vierwertiger Elemente" bezeichnet Oxide vierwertiger Elemente mit Ausnahme von SiO₂. Beispiele für weitere Oxide vierwertiger Elemente sind ZrO₂, SnO₂ und GeO₂ und insbesondere ZrO₂.

Der Begriff "weitere Oxide fünfwertiger Elemente" bezeichnet Oxide fünfwertiger Elemente mit Ausnahme von P₂O₅. Ein Beispiel für ein weiteres Oxid fünfwertiger Elemente ist Bi₂O₅.

Bevorzugt ist eine Glaskeramik, die mindestens ein weiteres Oxid vierwertiger Elemente oder ein weiteres Oxid fünfwertiger Elemente enthält.

Beispiele für Schmelzbeschleuniger sind Fluoride.

Beispiele für Färbemittel und Fluoreszenzmittel sind farbgebende oder fluoreszierende Oxide von d- und f-Elementen, wie z.B. die Oxide von Sc, Ti, Mn, Fe, Ag, Ce, Pr, Tb, Er und Yb, insbesondere Ti, Mn, Fe, Ag, Ce, Pr, Tb und Er.

Besonders bevorzugt ist eine Glaskeramik, die mindestens eine und bevorzugt alle folgenden Komponenten enthält:

| Komponente | Gew.-% |
|---|---|
| SiO₂ | 54,0 bis 80,0, insbesondere 60,0 bis 70,0 |
| Li₂O | 11,0 bis 19,0, insbesondere 12,0 bis 15,0 |
| K₂O | 0,5 bis 13,5, insbesondere 1,0 bis 7,0 |
| Al₂O₃ | 0,2 bis 8,0, insbesondere 1,0 bis 7,0 |
| Erdalkalioxid | 0 bis 6,0, insbesondere 0,1 bis 5,0 |
| ZnO | 0 bis 6,0, insbesondere 0,1 bis 5,0 |
| Übergangsmetalloxid | 8,5 bis 30,0, insbesondere 9,0 bis 25,0 |
| P₂O₅ | 0,5 bis 12,0, insbesondere 2,5 bis 6,0 |
| ZrO₂ | 0,1 bis 4,0, insbesondere 0,5 bis 2,0 |
| Färbemittel und Fluoreszenzmittel | 0,1 bis 8,0, insbesondere 0,2 bis 2,0. |

Der im Folgenden verwendete Begriff "Hauptkristallphase" bezeichnet die Kristallphase, die gegenüber anderen Kristallphasen den höchsten Volumenanteil hat.

Die erfindungsgemäß eingesetzte Glaskeramik weist bevorzugt Lithiummetasilikat als Hauptkristallphase auf. Insbesondere enthält die Glaskeramik mehr als 5 Vol.-%, bevorzugt mehr als 10 Vol.-% und besonders bevorzugt mehr als 15 Vol.-% an Lithiummetasilikat-Kristallen, bezogen auf die gesamte Glaskeramik.

In einer weiteren bevorzugten Ausführungsform weist die Glaskeramik Lithiumdisilikat als Hauptkristallphase auf. Insbesondere enthält die Glaskeramik mehr als 5 Vol.-%, bevorzugt mehr als 10 Vol.-% und besonders bevorzugt mehr als 15 Vol.-% an Lithiumdisilikat-Kristallen, bezogen auf die gesamte Glaskeramik.

Die erfindungsgemäß eingesetzte Lithiumdisilikat-Glaskeramik zeichnet sich durch besonders gute mechanische Eigenschaften aus und kann durch Wärmebehandlung der erfindungsgemäß eingesetzten Lithiummetasilikat-Glaskeramik erzeugt werden. Dies kann z.B. auch im Rahmen des erfindungsgemäßen Verpressens bei erhöhter Temperatur zur gewünschten Dentalrestauration erfolgen.

Auch ist es überraschend, dass die erfindungsgemäß eingesetzte Lithiumdisilikat-Glaskeramik trotz ihres hohen Gehalts an Oxid eines Übergangsmetalls mit hoher Ordnungszahl regelmäßig eine gute Transluzenz besitzt und bei ihr keine Amorph-Amorph-Phasentrennung auftritt.

Die erfindungsgemäß eingesetzte Lithiumdisilikat-Glaskeramik hat neben guten mechanischen Eigenschaften auch eine hohe chemische Beständigkeit.

Im erfindungsgemäßen Verfahren kann ebenfalls Lithiumsilikat-glas verwendet werden, das die Komponenten der oben beschriebenen erfindungsgemäßen Glaskeramik enthält und Keime enthält, die zur Ausbildung von Lithiummetasilikat- und/oder Lithiumdisilikatkristallen geeignet sind. Hinsichtlich bevorzugter Ausführungsformen dieses Glases wird auf die oben beschriebenen bevorzugten Ausführungsformen der erfindungsgemäßen Glaskeramik verwiesen. Es hat sich überraschend gezeigt, dass trotz des hohen Gehalts an Übergangsmetall mit hoher Ordnungszahl homogene, klare Gläser erhalten werden können, die keinerlei unerwünschte Phänomene wie Amorph-Amorph-Phasentrennung oder Spontankristallisation zeigen. Diese Gläser sind zur Herstellung der erfindungsgemäß eingesetzten Glaskeramik durch Wärmebehandlung geeignet.

Das erfindungsgemäß eingesetzte Glas mit Keimen kann durch Wärmebehandlung eines entsprechend zusammengesetzten Ausgangsglases erzeugt werden. Durch eine weitere Wärmebehandlung kann dann die erfindungsgemäße Lithiummetasilikat-Glaskeramik gebildet werden, die ihrerseits durch weitere Wärmebehandlung in die erfindungsgemäße Lithiumdisilikat-Glaskeramik umgewandelt werden kann. Mithin können das Ausgangsglas, das Glas mit Keimen und die Lithiummetasilikat-Glaskeramik als Vorstufen zur Erzeugung der hochfesten Lithiumdisilikat-Glaskeramik angesehen werden. Die zur Umwandlung erforderlichen Wärmebehandlungen können dabei auch im Rahmen des Verpressens bei erhöhter Temperatur erfolgen. Das würde dazu führen, dass das Verpressen nicht nur die Formung der gewünschten Dentalrestauration aus dem eingesetzten Glas oder der Glaskeramik bewirkt, sondern auch zu dessen Umwandlung, z.B. Glas mit Keimen umgewandelt zu Glaskeramik oder Lithiummetasilikat-Glaskeramik umgewandelt zu Lithiumdisilikat-Glaskeramik, führt.

Die eingesetzten Gläser und Glaskeramiken liegen üblicher Weise in Form von Rohlingen vor, da sie in dieser Form einfach im Rahmen des erfindungsgemäßen Verfahrens verarbeitet werden können.

Das Verfahren zur Herstellung der erfindungsgemäß eingesetzten Glaskeramik und des erfindungsgemäß eingesetzten Glases mit Keimen zeichnet sich dadurch aus, dass ein Ausgangsglas mit den Komponenten der Glaskeramik oder des Glases mindestens einer Wärmebehandlung im Bereich von 450 bis 950 °C unterzogen wird.

Das Ausgangsglas enthält daher mindestens 8,5 Gew.-% Oxid von mindestens einem Übergangsmetall wie oben definiert. Darüber hinaus enthält es bevorzugt auch geeignete Mengen an SiO₂ und Li₂O, um die Ausbildung einer Lithiumsilikat-Glaskeramik zu ermöglichen. Weiter kann das Ausgangsglas auch noch andere Komponenten enthalten, wie sie oben für die erfindungsgemäß eingesetzte Lithiumsilikat-Glaskeramik angegeben sind. Dabei sind solche Ausführungsformen bevorzugt, die auch für die Glaskeramik als bevorzugt angegeben sind.

Zur Herstellung des Ausgangsglases wird insbesondere so vorgegangen, dass eine Mischung von geeigneten Ausgangsmaterialien, wie z.B. Carbonaten, Oxiden, Phosphaten und Fluoriden, bei Temperaturen von insbesondere 1300 bis 1600 °C, vorzugsweise 1450 bis 1500 °C, für 2 bis 10 h erschmolzen wird. Zur Erzielung einer besonders hohen Homogenität wird die erhaltene Glasschmelze in Wasser gegossen, um ein Glasgranulat zu bilden, und das erhaltene Granulat wird dann erneut aufgeschmolzen.

Die Schmelze kann dann in Formen gegossen werden, um Rohlinge des Ausgangsglases, sogenannte Massivglasrohlinge oder monolithische Rohlinge, zu erzeugen. Vorzugsweise erfolgt die Abkühlung ab einer Temperatur von 500 °C mit einer Abkühlgeschwindigkeit von 3 bis 5 K/min bis auf Raumtemperatur. Dies ist insbesondere zur Erzeugung spannungsfreier Glasprodukte vorteilhaft.

Es ist ebenfalls möglich, die Schmelze erneut in Wasser zu geben, um ein Granulat herzustellen. Dieses Granulat kann dann nach Mahlen und gegebenenfalls Zugabe weiterer Komponenten, wie Färbe- und Fluoreszenzmitteln, zu einem Rohling, einem sogenannten Pulverpressling, gepresst werden.

Schließlich kann das Ausgangsglas nach Granulierung auch zu einem Pulver verarbeitet werden.

Anschließend wird das Ausgangsglas, z.B. in Form eines Massivglasrohlings, eines Pulverpresslings oder in Form eines Pulvers, mindestens einer Wärmebehandlung im Bereich von 450 bis 950 °C unterzogen. Es ist bevorzugt, dass zunächst bei einer Temperatur im Bereich von 500 bis 600 °C eine erste Wärmebehandlung durchgeführt wird, um ein Glas mit Keimen herzustellen, welche zur Bildung von Lithiummetasilikat- und/oder Lithiumdisilikat-Kristallen geeignet sind. Dieses Glas kann dann bevorzugt mindestens einer weiteren Temperaturbehandlung bei einer höheren Temperatur und insbesondere mehr als 570 °C unterworfen werden, um Kristallisation von Lithiummetasilikat oder von Lithiumdisilikat zu bewirken.

Diese mindestens eine Wärmebehandlung kann auch im Rahmen des erfindungsgemäßen Verfahrens beim Verpressen bei erhöhter Temperatur erfolgen. Gleichfalls ist es möglich, nach der maschinellen Bearbeitung das eingesetzte Glas oder die eingesetzte Glaskeramik durch diese Wärmebehandlung umzuwandeln und insbesondere zu hochfester Lithiumdisilikat-Glaskeramik umzuwandeln.

Aus der erfindungsgemäß eingesetzten Glaskeramik und dem erfindungsgemäß eingesetzten Glas können dentale Restaurationen, wie Inlays, Onlays, Kronen, Veneers, Schalen oder Abutments, hergestellt werden. Die Erfindung betrifft daher auch deren Verwendung zur Herstellung dentaler Restaurationen. Dabei ist es bevorzugt, dass die Glaskeramik oder das Glas durch Verpressen oder maschinelle Bearbeitung zur gewünschten dentalen Restauration verformt wird. Das Verpressen erfolgt üblicherweise unter erhöhtem Druck, von z.B. 2 bis 10 bar, und erhöhter Temperatur, von z.B. 700 bis 1200°C. Hierzu können insbesondere die in der EP 231 773 beschriebenen Verfahren und der dort offenbarte Pressofen eingesetzt werden. Ein geeigneter Ofen ist z.B. der Programat EP 5000 von Ivoclar Vivadent AG, Liechtenstein. Es können für das Verpressen vor allem das erfindungsgemäße Ausgangsglas und insbesondere das erfindungsgemäße Glas mit Keimen, die erfindungsgemäße Lithiummetasilikat- und die erfindungsgemäße Lithiumdisilikat-Glaskeramik in geeigneter Weise, z.B. in Form von Rohlingen, eingesetzt werden.

Die maschinelle Bearbeitung wird üblicherweise im Rahmen eines CAD/CAM-Verfahrens durchgeführt, und sie nutzt insbesondere die erfindungsgemäße Lithiummetasilikat- und Lithiumdisilikat-Glaskeramik, vorzugsweise in Form von geeigneten Rohlingen. Die maschinelle Bearbeitung erfolgt dabei insbesondere mittels materialabtragender Verfahren, wie Fräsen und/oder Schleifen.

Nach der Herstellung der gewünscht geformten dentalen Restauration durch Verpressen oder maschinelle Bearbeitung kann diese insbesondere noch wärmebehandelt werden, um eingesetzte Vorläufer, wie Ausgangsglas, Glas mit Keimen oder Lithiummetasilikat-Glaskeramik, in Lithiumdisilikat-Glaskeramik umzuwandeln.

Die erfindungsgemäß eingesetzten Gläser und Glaskeramiken können schließlich auch zusammen mit anderen Gläsern und Glaskeramiken gemischt werden, um Dentalmaterialien mit gewünscht eingestellten Eigenschaften zu ergeben. Ein Glas oder eine Glaskeramik, die das erfindungsgemäße Glas oder die erfindungsgemäße Glaskeramik enthalten, stellen daher einen weiteren Gegenstand der Erfindung dar. Das erfindungsgemäße Glas oder die erfindungsgemäße Glaskeramik können daher insbesondere als Hauptkomponente eines anorganisch-anorganischen Komposits oder in Kombination mit einer Vielzahl von anderen Gläsern und/oder Glaskeramiken verwendet werden, wobei die Komposite oder Kombinationen insbesondere als Dentalmaterialien eingesetzt werden können. Besonders bevorzugt können die Kombinationen oder Komposite in Form von Sinterrohlingen vorliegen. Beispiele anderer Gläser und Glaskeramiken zur Herstellung anorganisch-anorganischer Komposite und von Kombinationen sind in DE 43 14 817, DE 44 23 793, DE 44 23 794, DE 44 28 839, DE 196 47 739, DE 197 25 552 und DE 100 31 431 offenbart. Diese Gläser und Glaskeramiken gehören zur Silikat-, Borat-, Phosphat- oder Alumosilikat-Gruppe. Bevorzugte Gläser und Glaskeramiken sind vom SiO₂-Al₂O₃-K₂O-Typ (mit kubischen oder tetragonalen Leucit-Kristallen), SiO₂-B₂O₃-Na₂O-Typ, Alkali-Silikat-Typ, Alkali-Zink-Silikat-Typ, Silico-Phosphat-Typ und/oder SiO₂-ZrO₂-Typ. Durch Vermischen derartiger Gläser oder Glaskeramiken mit den erfindungsgemäßen Gläsern und/oder Glaskeramiken kann beispielsweise der Wärmeausdehnungskoeffizient in einem breiten Bereich von 6 bis 20 • 10⁻⁶ K⁻¹ in gewünschter Weise eingestellt werden.

Die Erfindung wird im Folgenden anhand von Beispielen näher erläutert.

### Beispiele

### Beispiele 1 bis 10 - Zusammensetzung und Kristallphasen

Es wurden insgesamt 10 Gläser und Glaskeramiken mit der in Tabelle I angegebenen Zusammensetzung (jeweils in Gew.-%) über Erschmelzung entsprechender Ausgangsgläser und anschließende Wärmebehandlung zur gesteuerten Keimbildung und Kristallisation hergestellt.

Die Ausgangsgläser wurden zunächst im 100 bis 200 g Maßstab aus üblichen Rohstoffen bei 1400 bis 1500 °C erschmolzen und durch Eingießen in Wasser in Glasfritten umgewandelt. Diese Glasfritten wurden zur Homogenisierung anschließend ein zweites Mal bei 1450 bis 1550 °C für 1 bis 3 h geschmolzen. Die erhaltenen Glasschmelzen wurden in vorgewärmte Formen gegossen, um Glasmonolithe zu erzeugen. Diese Glasmonolithe wurden durch thermische Behandlung zu erfindungsgemäßen Gläsern und Glaskeramiken umgewandelt.

Die nach Abschluss aller Wärmebehandlungen erhaltenen Kristallphasen wurden durch Hochtemperatur-Röntgenbeugung (HT-XRD) bei den jeweils in Tabelle I aufgeführten Temperaturen ermittelt. Es wurden überraschenderweise stets Glaskeramiken mit Lithiumdisilikat als Hauptkristallphase erhalten. Trotz des hohen Gehaltes an Übergangsmetallen mit hoher Ordnungszahl wurden keine Nebenkristallphasen mit diesen Übergangsmetallen gefunden.

Schließlich wurden die Brechungsindizes der jeweiligen Glasphasen mittels Abbe-Refraktometrie (20 °C, 589 nm) bestimmt. Dabei zeigte sich, dass die erfindungsgemäßen Glaskeramiken einen deutlich höheren Brechungsindex als die Vergleichs-Glaskeramik aufwiesen.

**Tabelle I**

| | **1** | **2** | **3** | **4** | **5** | **6** | **7** | **8** | **9** | **10** |
|---|---|---|---|---|---|---|---|---|---|---|
| **SiO₂** | 67,4 | 58,4 | 66,4 | 63,5 | 67,0 | 61,8 | 66,4 | 66,4 | 61,8 | 54,5 |
| **K₂O** | 3,7 | 1,0 | 2,9 | 2,8 | 2,9 | 1,0 | 2,9 | 2,9 | 1,0 | 0,5 |
| **Li₂O** | 14,1 | 12,1 | 13,8 | 13,2 | 14,4 | 13,2 | 13,8 | 13,8 | 13,2 | 11,3 |
| **Al₂O₃** | 3,2 | 1,0 | 2,9 | 2,5 | | 1,0 | 2,9 | 2,9 | 1,0 | 0,5 |
| **P₂O₅** | 3,1 | 2,5 | 4,0 | 4,0 | 4,0 | 5,0 | 4,0 | 4,0 | 5,0 | 3,2 |
| **WO₃** | 8,5 | | | | | | | | | |
| **Nb₂O₅** | | | 10,0 | | | | | | | |
| **Ta₂O₅** | | | | | | | 10,0 | | | |
| **La₂O₃** | | 25, 0 | | | | | | 10, 0 | 18, 0 | 30,0 |
| **Y₂O₃** | | | | 14,0 | 10,0 | 18,0 | | | | |
| **CeO₂** | | | | | 1,0 | | | | | |
| **Er₂O₃** | | | | | 0, 3 | | | | | |
| **Tb₄O₇** | | | | | 0,4 | | | | | |
| **Summe** | 100,0 | 100,0 | 100,0 | 100,0 | 100,0 | 100,0 | 100,0 | 100,0 | 100,0 | |
| **Kristallphase (n) HT-XRD** | Li₂Si₂O₅ (800°C) | | Li₂Si₂O₅ (800°C) | Li₂Si₂O₅ KAlSiO₄ (820°C) | Li₂Si₂O₅ (780°C) | Li₂Si₂O₅ Li₂SiO₃ (800°C) | Li₂Si₂O₅ (800°C) | Li₂Si₂O₅ Li₂SiO₃ (700°C) | Li₂Si₂O₅ LaPO₄ (800°C) | |
| **Brechungs index n_{d}** | 1,5312 | | 1,5547 | 1,5553 | 1,5494 | 1,5643 | 1,5403 | 1,5422 | 1,5586 | |

### Beispiel 11 - Direkte Herstellung dentaler Restaurationen durch Heißpressen oder maschinelle Bearbeitung (CAD/CAM)

### (A) Glasrohlinge mit Keimen

Es wurden zunächst Gläser mit der Zusammensetzung gemäß Beispielen 7 und 8 hergestellt, indem entsprechende Rohstoffe in Form von Oxiden und Carbonaten 30 min in einem Turbola-Mischer gemischt und anschließend bei 1450°C für 120 min in einem Platintiegel erschmolzen wurden. Die Schmelzen wurden in Wasser gegossen, um feinteilige Glasgranulate zu erhalten. Diese Glasgranulate wurden erneut bei 1530°C für 150 min geschmolzen, um Glasschmelzen mit besonders hoher Homogenität zu erhalten. Die Temperatur wurde für 30 min auf 1500°C abgesenkt und anschließend wurden a) quaderförmige (12.5mm x 14mm x 40mm) und b) zylindrische Glasrohlinge mit einem Durchmesser von 12.5mm in vorgeheizte, teilbare Stahlformen oder Graphitformen gegossen. Danach wurden die erhaltenen Glasquader oder Glaszylinder im Bereich von 500-560°C je nach Zusammensetzung wärmebehandelt, um Keime für Lithiummetasilikat- und/oder Lithiumdisilikat-Kristalle auszubilden und das Glas zu entspannen.

Die erhaltenen Rohlinge mit Keimen wurden gemäß der folgenden Alternativen zu Restaurationen weiterverarbeitet.

### (B) Heißpressen von Glas mit Keimen, Lithiummetasilikat- oder Lithiumdisilikat-Glaskeramik

i) Die Glaszylinder mit Keimen (A) wurden durch Heißpressen bei einer Presstemperatur von 900-950°C unter Verwendung eines Pressofens EP600, Ivoclar Vivadent AG, zu dentalen Restauration, wie Inlays, Onlays, Veneers, Teilkronen, Kronen und Schalen, verarbeitet. Als Hauptkristallphase konnte in den Restaurationen Lithiumdisilikat nachgewiesen werden.
ii) Die Glaszylinder mit Keimen (A) wurden einer ersten Kristallisation bei 650 bis 750°C für 20 min unterworfen. Dabei betrug die Aufheizgeschwindigkeit 15°C pro Minute. Nach dieser ersten Kristallisation konnte als Hauptkristallphase Lithiummetasilikat nachgewiesen werden. Durch Heißpressen der Lithiummetasilikat-Glaszylinder bei einer Presstemperatur von 900-950°C unter Verwendung eines Pressofens EP600, Ivoclar Vivadent AG, konnten dentalen Restauration, wie Inlays, Onlays, Veneers, Teilkronen, Kronen und Schalen, hergestellt werden. Dabei wurde durch das Heißpressen Lithiummetasilikat in Lithiumdisilikat umgewandet.
iii) Die Glaszylinder mit Keimen (A) wurden nach einer ersten Kristallisation gemäß ii) zusätzlich einer weiteren thermischen Behandlung bei 840 bis 880°C für 5 bis 30 min. unterworfen. Die Kristallphasenanalyse zeigte nach dieser Behandlung eine erfindungsgemäße Glaskeramik mit Lithiumdisilikat als Hauptkristallphase. Die nach der zweiten Kristallisation erhaltenen kristallisierten Zylinder wurden anschließend durch Heißpressen bei einer Presstemperatur von 900-950°C unter Verwendung eines Pressofens EP600, Ivoclar Vivadent AG, zu dentalen Restaurationen, wie Inlays, Onlays, Veneers, Kronen, Teilkronen und Schalen, weiterverarbeitet.

### (C) Maschinelle Bearbeitung (CAD/CAM-Verfahren) von Lithiummetasilikat

Die Glasquader mit Keimen (A) wurden zunächst einer ersten Kristallisation gemäß (B) (ii) unterzogen, um die Kristallisation von Lithiummetasilikat zu bewirken. Aus den erhaltenen Lithiummetasilikat-Glasquadern wurden dann mittels CAD/CAM-Verfahren, z.B. Sirona, Cerec 2^{®} oder Cerec 3^{®}, maschinell dentale Restaurationen, wie Inlays, Onlays, Kronen, Teilkronen, Schalen und Veeners, herausgearbeitet. Anschließend wurden diese Restaurationen einer zweiten Kristallisation bei 840 bis 880°C für 5 min bis 1 h unterworfen. Die Kristallphasenanalyse zeigte nach dieser Behandlung eine erfindungsgemäße Glaskeramik mit Lithiumdisilikat als Hauptkristallphase.

## Patentansprüche

1. Verfahren zur Herstellung von Dentalrestaurationen, bei dem eine Lithiumsilikat-Glaskeramik oder ein Lithiumsilikat-Glas durch
(i) Verpressen oder
(ii) maschinelle Bearbeitung
zur Dentalrestauration verformt wird, wobei die Glaskeramik und das Glas mindestens 8,5 Gew.-% Übergangsmetalloxid ausgewählt aus der Gruppe bestehend aus Oxiden von Y, Nb, La, Ta, W und Mischungen dieser Oxide enthalten und wobei die Glaskeramik Lithiummetasilikat oder Lithiumdisilikat als Hauptkristallphase aufweist und das Glas Keime enthält, die zur Ausbildung von Lithiummetasilikat- und/oder Lithiumdisilikatkristallen geeignet sind.

2. Verfahren nach Anspruch 1, bei dem die Dentalrestaurationen ausgewählt sind aus Inlays, Onlays, Kronen, Veneers, Schalen oder Abutments.

3. Verfahren nach Anspruch 1 oder 2, bei dem das Verpressen bei einer Temperatur von 700 bis 1200°C erfolgt.

4. Verfahren nach einem der Ansprüche 1 bis 3, bei dem das Verpressen bei einem Druck von 2 bis 10 bar erfolgt.

5. Verfahren nach einem der Ansprüche 1 bis 4, bei dem die maschinelle Bearbeitung im Rahmen eines CAD/CAM-Verfahrens erfolgt.

6. Verfahren nach einem der Ansprüche 1 bis 5, bei dem die Glaskeramik Lithiummetasilikat als Hauptkristallphase aufweist und das Glas Keime enthält, die zur Ausbildung von Lithiummetasilikatkristallen geeignet sind.

7. Verfahren nach einem der Ansprüche 1 bis 5, bei dem die Glaskeramik Lithiumdisilikat als Hauptkristallphase aufweist und das Glas Keime enthält, die zur Ausbildung von Lithiumdisilikatkristallen geeignet sind.

8. Verfahren nach einem der Ansprüche 1 bis 7, bei dem die Glaskeramik oder das Glas mindestens eine und bevorzugt alle folgenden Komponenten enthält:
| Komponente | Gew.-% |
|---|---|
| SiO₂ | 54,0 bis 80,0, insbesondere 60,0 bis 70,0 |
| Li₂O | 11,0 bis 19,0, insbesondere 13,0 bis 17,0 |
| Al₂O₃ | 0,2 bis 8,0, insbesondere 1,0 bis 7,0 |
| K₂O | 0,5 bis 13,5, insbesondere 1,0 bis 7,0 |
| Erdalkalioxid | 0 bis 6,0, insbesondere 0,1 bis 5,0 |
| ZnO | 0 bis 6,0, insbesondere 0,1 bis 5,0 |
| Übergangsmetalloxid | 8,5 bis 30,0, insbesondere 9,0 bis 25,0 |
| P₂O₅ | 0,5 bis 12,0, insbesondere 2,5 bis 6,0 |
| ZrO₂ | 0,1 bis 4,0, insbesondere 0,5 bis 2,0 |
| Färbemittel und Fluoreszenzmittel | 0,1 bis 8,0, insbesondere 0,2 bis 2,0. |

9. Verfahren nach einem der Ansprüche 1 bis 8, bei dem die Glaskeramik und das Glas in Form von Rohlingen eingesetzt werden.

10. Verfahren nach einem der Ansprüche 1 bis 9, bei dem nach der maschinellen Bearbeitung mindestens eine Wärmebehandlung erfolgt, um das verformte Glas oder die verformte Glaskeramik zu Lithiumdisilikat-Glaskeramik umzuwandeln.

11. Verfahren nach einem der Ansprüche 1 bis 10, bei dem die Dentalrestauration auf Basis von Lithiumdisilikat-Glaskeramik ist.

12. Verwendung einer Lithiumsilikat-Glaskeramik oder eines Lithiumsilikat-Glases zur Herstellung von Dentalrestaurationen, wobei die Glaskeramik oder das Glas durch
(i) Verpressen oder
(ii) maschinelle Bearbeitung
zur Dentalrestauration verformt wird, wobei die Glaskeramik und das Glas mindestens 8,5 Gew.-% Übergangsmetalloxid ausgewählt aus der Gruppe bestehend aus Oxiden von Y, Nb, La, Ta, W und Mischungen dieser Oxide enthalten und wobei die Glaskeramik Lithiummetasilikat oder Lithiumdisilikat als Hauptkristallphase aufweist und das Glas Keime enthält, die zur Ausbildung von Lithiummetasilikat- und/oder Lithiumdisilikatkristallen geeignet sind.

## Claims

1. Process for the preparation of dental restorations, wherein a lithium silicate glass ceramic or a lithium silicate glass is shaped to the dental restoration by
(i) pressing or
(ii) machining,
wherein the glass ceramic and the glass comprise at least 8.5 wt.-% transition metal oxide selected from the group consisting of oxides of Y, Nb, La, Ta, W and mixtures of these oxides and wherein the glass ceramic comprises lithium metasilicate or lithium disilicate as main crystal phase and the glass comprises nuclei suitable for forming lithium metasilicate and/or lithium disilicate crystals.

2. Process according to claim 1, wherein the dental restorations are selected from inlays, onlays, crowns, veneers, facets and abutments.

3. Process according to claim 1 or 2, wherein the pressing is conducted at a temperature of 700 to 1200°C.

4. Process according to any one of claims 1 to 3, wherein the pressing is conducted at a pressure of 2 to 10 bar.

5. Process according to any one of claims 1 to 4, wherein the machining is effected in a CAD/CAM process.

6. Process according to any one of claims 1 to 5, wherein the glass ceramic comprises lithium metasilicate as main crystal phase and the glass comprises nuclei suitable for forming lithium metasilicate crystals.

7. Process according to any one of claims 1 to 5, wherein the glass ceramic comprises lithium disilicate as main crystal phase and the glass comprises nuclei suitable for forming lithium disilicate crystals.

8. Process according to any one of claims 1 to 7, wherein the glass ceramic or the glass comprises at least one and preferably all of the following components:
| Component | wt.-% |
|---|---|
| SiO₂ | 54.0 to 80.0, in particular 60.0 to 70.0 |
| Li₂O | 11.0 to 19.0, in particular 13.0 to 17.0 |
| Al₂O₃ | 0.2 to 8.0, in particular 1.0 to 7.0 |
| K₂O Alkaline earth | 0.5 to 13.5, in particular 1.0 to 7.0 |
| oxide | 0 to 6.0, in particular 0.1 to 5.0 |
| ZnO | 0 to 6.0, in particular 0.1 to 5.0 |
| Transition metal oxide | 8.5 to 30.0, in particular 9.0 to 25.0 |
| P₂O₅ | 0.5 to 12.0, in particular 2.5 to 6.0 |
| ZrO₂ | 0.1 to 4.0, in particular 0.5 to 2.0 |
| Colorant and fluorescent agent | 0.1 to 8.0, in particular 0.2 to 2.0. |

9. Process according to any one of claims 1 to 8, wherein the glass ceramic and the glass are used in the form of blanks.

10. Process according to any one of claims 1 to 9, wherein after the machining at least one heat treatment is conducted to convert the shaped glass or the shaped glass ceramic to lithium disilicate glass ceramic.

11. Process according to any one of claims 1 to 10, wherein the dental restoration is on the basis of lithium disilcate glass ceramic.

12. Use of a lithium silicate glass ceramic or a lithium silicate glass for preparing dental restorations, wherein the glass ceramic or the glass is shaped to the dental restoration by
(i) pressing or
(ii) machining, and
wherein glass ceramic and the glass comprise at least 8.5 wt.-% transition metal oxide selected from the group consisting of oxides of Y, Nb, La, Ta, W and mixtures of these oxides and wherein the glass ceramic comprises lithium metasilicate or lithium disilicate as main crystal phase and the glass comprises nuclei suitable for forming lithium metasilicate and/or lithium disilicate crystals.

## Revendications

1. Procédé de fabrication de restaurations dentaires, selon lequel une vitrocéramique à base de silicate de lithium ou un verre à base de silicate de lithium est mis(e) en forme par
(i) pressage ou
(ii) façonnage mécanique
pour obtenir la restauration dentaire, la vitrocéramique et le verre contenant au moins 8,5 % en poids d'oxyde de métal de transition, sélectionné dans le groupe comprenant des oxydes de Y, Nb, La, Ta, W et des mélanges de ces oxydes, et la vitrocéramique présentant du métasilicate de lithium ou du disilicate de lithium comme phase cristalline principale, et le verre contenant des germes qui conviennent pour la formation de cristaux de métasilicate de lithium et/ou de disilicate de lithium.

2. Procédé selon la revendication 1, selon lequel les restaurations dentaires sont sélectionnées parmi des inlays, des onlays, des couronnes, des facettes dentaires, des coques ou des piliers.

3. Procédé selon la revendication 1 ou 2, selon lequel le pressage est réalisé à une température allant de 700 à 1 200 ˙˙C.

4. Procédé selon l'une des revendications 1 à 3, selon lequel le pressage est réalisé à une pression allant de 2 à 10 bars.

5. Procédé selon l'une des revendications 1 à 4, selon lequel le façonnage mécanique est réalisé dans le cadre d'un procédé CAO/FAO.

6. Procédé selon l'une des revendications 1 à 5, selon lequel la vitrocéramique présente du métasilicate de lithium comme phase cristalline principale, et le verre contient des germes qui conviennent pour la formation de cristaux de métasilicate de lithium.

7. Procédé selon l'une des revendications 1 à 5, selon lequel la vitrocéramique présente du disilicate de lithium comme phase cristalline principale, et le verre contient des germes qui conviennent pour la formation de cristaux de métasilicate de lithium.

8. Procédé selon l'une des revendications 1 à 7, selon lequel la vitrocéramique ou le verre contient au moins un et de préférence tous les constituants suivants :
| Constituant | % en poids |
|---|---|
| SiO₂ | 54,0 à 80,0, notamment 60,0 à 70,0 |
| Li₂O | 11,0 à 19,0, notamment 13,0 à 17,0 |
| Al₂O₃ | 0,2 à 8,0, notamment 1,0 à 7,0 |
| K₂O | 0,5 à 13,5, notamment 1,0 à 7,0 |
| Oxyde alcalino-terreux | 0,0 à 6,0, notamment 0,1 à 5,0 |
| ZnO | 0,0 à 6,0, notamment 0,1 à 5,0 |
| Oxyde de métal de transition | 8,5 à 30,0, notamment 9,0 à 25,0 |
| P₂O₅ | 0,5 à 12,0, notamment 2,5 à 6,0 |
| ZrO₂ | 0,1 à 4,0, notamment 0,5 à 2,0 |
| Colorant et agent fluorescent | 0,1 à 8,0, notamment 0,2 à 2,0 |

9. Procédé selon l'une des revendications 1 à 8, selon lequel la vitrocéramique et le verre sont utilisés sous forme d'ébauches.

10. Procédé selon l'une des revendications 1 à 9, selon lequel le façonnage mécanique est suivi d'au moins un traitement thermique pour transformer le verre mis en forme ou la vitrocéramique mise en forme en vitrocéramique au disilicate de lithium.

11. Procédé selon l'une des revendications 1 à 10, selon lequel la restauration dentaire est à base de vitrocéramique au disilicate de lithium.

12. Utilisation d'une vitrocéramique à base de silicate de lithium ou d'un verre à base de silicate de lithium pour la fabrication de restaurations dentaires, où la vitrocéramique ou le verre est mis(e) en forme par
(i) compression ou
(ii) façonnage mécanique
pour obtenir la restauration dentaire, la vitrocéramique et le verre contenant au moins 8,5 % en poids d'oxyde de métal de transition, sélectionné dans le groupe comprenant des oxydes de Y, Nb, La, Ta, W et des mélanges de ces oxydes, et la vitrocéramique présentant du métasilicate de lithium ou du disilicate de lithium comme phase cristalline principale, et le verre contient des germes qui conviennent pour la formation de cristaux de métasilicate de lithium et/ou de disilicate de lithium.
